# EUROPEAN PATENT SPECIFICATION

(11) **EP 0 875 249 B1**
(45) Date of publication and mention of the grant of the patent: **12.03.2003**
(21) Application number: 98107887.6
(22) Date of filing: 28.04.1998
(51) Int. Cl.: A61K 33/26, A61K 38/38, A61K 38/40, A61K 47/26, A61P 7/06

(54) **Complexes consisting of FE(III), a polyhydroxylated compound, and ovalbumin**
Komplexe bestehend aus FE(III), Polyhydroxylverbindungen, und Ovalbumin
Complexes constitués de FE(III), d'un composé polyhydroxylé, et d'ovalbumine

(30) Priority: 30.04.1997 IT MI971012
(43) Date of publication of application: 04.11.1998
(73) Proprietor: LABORATORI DERIVATI ORGANICI S.P.A., 20122 Milano (IT)
(72) Inventor: Gonella, Sergio, 13048 Santhia, Vercelli (IT)
(74) Representative: Gervasi, Gemma, Dr.

(56) References cited:
- EP-A- 0 537 634
- WO-A-91/07426
- GB-A- 2 115 821
- CHEMICAL ABSTRACTS, vol. 124, no. 15, 9 April 1996 Columbus, Ohio, US; abstract no. 203109, XP002049397 & JP 07 304 798 A (SNOW BRAND MILK PROD. CO. LTD.,JP) 21 November 1995
- CHEMICAL ABSTRACTS, vol. 113, no. 13, 24 September 1990 Columbus, Ohio, US; abstract no. 111976, XP002049398 & JP 02 001 500 A (Q.P. CORP.,JP) 5 January 1990
- OWEN R. FENNEMA: "PRINCIPLES OF FOOD SCIENCE, Food chemistry, Part I, p.665-666", 1976, MARCEL DEKKER, NEW YORK, BASEL

## Description

### Prior art

Iron plays a fundamental physiological role in human metabolism.

The iron necessary in metabolism comes from absorption through the diet.

There are multiple factors influencing iron absorption: intraluminal factors (intestinal processes and secretions), state of the intestinal mucous membrane and bodily factors (iron turnover, erythropoiesis).

Iron loss due to menstruation and pregnancy, dietary deficiency, malabsorption in small children and the elderly due to alterations of the above-mentioned factors lead to iron deficiency which frequently bring about serious clinical situations.

Usually, if patients affected by sideropenic anaemia are administered martial therapy, the symptoms due to iron deficiency disappear and, provided that the therapeutic dose of iron is adequate, the mean haemoglobin increases day by day. However, in the majority of cases it is not sufficient to proceed with the martial therapy until normal haemoglobin levels are reached, but it is necessary to continue the therapy for several years until the anaemia is corrected.

The result is that in general the martial therapy must be continued for some time and, in not particularly serious cases, oral administrations based on iron salts are given, for example inorganic salts (ferrous sulphate), or else organic salts (citrate, fumarate, gluconate, etc.). At this point, there arises the problem of intolerance and toxicity which many iron salts cause at a gastro-intestinal level, above all in the case of inorganic salts.

In addition to iron salts, also iron complexes have been proposed, which have fewer side effects than do the salts, but present the disadvantage that they are less effective from a physiological point of view. In actual fact, in some complexes the iron can be chelated so strongly that its physiological action is very low (Remington's Pharmaceutical Science (1990), Pages 826-828).

Examples of these complexes consist of Fe(III)-polyol amino acids, as described in "Preparation and Properties of Iron(III)-L-Amino Acid Nitrates" (Inorganica Chimica Acta, 54 (1981), 187- 190) and in "Preparation and Properties of Iron(III)-Amino Acid Complexes Iron(III)-Alanine, a possible Ferritin Analog" (J. American Chem. Soc. 96, 2621 (1974).

In these complexes, the co-ordination takes place between oxides-hydroxides of Fe(III) and hydroxyls, and leads to compact structures based on a tetrahedral unit and stabilized by the presence of carboxyl side groups or other electrophilic groups.

Other iron complexes with proteins are rather unstable, unhomogeneous, not very soluble, and of low iron content.

Iron complexes of conalbumin with an iron glucide derivative such as iron saccharate or iron fructate, useful for the treatment of sideropenic and hypochronic anaemia, are disclosed by EP 0 537 634.

A considerable progress was made with the use of ferritin extracted from the spleen of horses or cows (Isolation, purification and characterization of Bovine Spleen Ferritin. N. Cetinkaya; F.W. Lengemann and P. Kogan; Comp. Biochem. Physiol. Vol. 80, No. 4, p. 773-78; 1985).

Ferritin does not cause damage to the gastro-intestinal walls but, since it is not very readily available, it has a very high cost. In addition, there exist problems linked to the use of bovine organs, which are potentially transmitters of non-conventional slow viruses, such as that causing BSE.

### Summary

Now a process has been found that enables complexes consisting of Fe(III), a polyhydroxylate compound and ovalbumin to be obtained, which have chemical and biological characteristics similar to the natural complexes of a ferritin type with high absorption capacity and exempt from side effects.

The above-mentioned process is characterized by the following operations:
A) Preparation of an aqueous solution of ovalbumin starting from an aqueous solution of albumin, in which the said albumin solution is treated with ammonium sulphate with a degree of saturation of 32%, to obtain a precipitate that is eliminated and a solution that is treated with ammonium sulphate with 52% degree of saturation, thus obtaining the precipitation of the ovalbumin, which is re-dissolved in water, purified, concentrated at approximately 5%, and to which is added NaOH to obtain a pH of 9;
B) Preparation of a solution of a complex consisting of Fe(III) and a polyhydroxylate compound in which to an aqueous solution of Mohr's salt is added the polyhydroxylate compound, and subsequently H₂O₂ is added to oxidize Fe(II) to Fe(III), and NaOH is added to the solution obtained up to a pH of 10.5;
C) Preparation of the Fe(III)-polyhydroxylate compound-ovalbumin complex by mixing the solutions obtained as described in points A) and B) and precipitating the complex by adding acetic acid up to a pH of between 5.0 and 5.5, followed by purification.

The complexes according to the present invention may be used as active substances in the therapy of sideropenic anaemia.

Said complexes are characterized by a high degree of homogeneity and bioavailability.

### Detailed description of the invention

The characteristics and advantages of the complexes according to the present invention and of the corresponding preparation process will be illustrated in greater detail in the course of the ensuing description.

The process for preparing the complexes consisting of Fe(III), a polyhydroxylate compound and ovalbumin according to the present invention is carried out by means of the following operations:
A) Preparation of a solution of ovalbumin;
B) Preparation of a solution of a complex consisting of Fe(III) and a polyhydroxylate compound;
C) Reaction between the solution of operation A) and the solution of operation B).

A) The ovalbumin solution is prepared using as raw material fresh albumin or flaked albumin and proceeding according to the following steps:
   A₁) The albumin is dissolved in water at a concentration of between 2 and 5 wt% at a temperature of between 5 and 10°C with moderate stirring, and an inorganic acid, such as phosphoric acid or sulphuric acid is added to the solution to obtain a pH of between 3 and 5.
   A₂) To the solution of stage A₁ is added ammonium sulphate in a quantity such as to obtain a degree of saturation of 32%, obtaining the precipitation of the proteins having molecular weights higher than that of ovalbumin.
      The said proteins are separated by means of decantation followed by filtration.
   A₃) To the solution obtained from the filtration described in step A₂ is added ammonium sulphate in a quantity such as to obtain a degree of saturation of 52%, obtaining the precipitation of the ovalbumin, whilst the proteins having a molecular weight lower than that of the ovalbumin remain in solution. The mixture is filtered, and the ovalbumin collected on the filter is washed with an ammonium sulphate solution until there is a negative reaction of the components reactive to the trichloro-acetic acid.
   A₄) The ovalbumin is dissolved in water at a concentration of 2 wt%. The solution then undergoes filtration and ultrafiltration with molecular fractionation of 20,000 until there is total elimination of the organic impurities and the ammonium sulphate.

The solution is concentrated up to approximately 5 wt% of albumin and is brought up to pH 9 by the addition of an NaOH solution.
B) The solution of the complex consisting of Fe(III) and a polyhydroxylate compound is prepared using as raw materials the ferrous ammonium sulphate (Mohr's salt) and a polyhydroxylate compound chosen from between mannitol, sorbitol and fructose, and proceeding according to the following steps:
   B₁) The Mohr's salt is dissolved in water at a temperature of between 10 and 15°C and in a quantity such as to obtain a concentration of between 3 and 5.25 wt%.

To the solution thus obtained is added the polyhydroxylate compound in the quantity of 0.5 mol. per 2.5 mol. of Mohr's salt.

The reaction is immediate, and a clear, practically colourless solution is obtained, with a pH of between 4 and 4.5. The solution is kept stirred at a temperature of between 10 and 15°C for 30 minutes.
B₂) Subsequently, 36% H₂O₂ is added slowly and in a quantity of 0.25 mol. per 2.5 mol. of Mohr's salt.

The Fe(II) is oxidized to Fe(III), and the solution turns a dark red colour, with a drop in the pH to values of between 2.5 and 2.7.

The solution is kept stirred and heated until a temperature of approximately 30-35°C is reached, and is kept at this temperature for 15-20 minutes.
B₃) To the solution referred to in point B₂ is gradually added a solution of 20 wt% NaOH in the quantity of 0.5 mol. per 2.5 mol. of Mohr's salt, to reach a pH of 10.5.

Initially, a precipitate is formed, which is dissolved as the NaOH solution is added. The reaction is completed by heating up to 80°C for approximately one hour with the total dissolution of the Fe(III)-polyhydroxylate compound.

The solution is diluted with an equal volume of water and kept stirred at room temperature for 10-15 hours.

The solution is filtered and then purified by being passed through molecular fractionation (1,000) columns to eliminate the free iron.
C) The formation of the Fe(III)Fe(III)-polyhydroxylate compound-ovalbumin complex is obtained by proceeding according to the following steps:
   C₁) The ovalbumin solution obtained from the operation described in A) is mixed with the solution of the Fe(III)Fe(III)-polyhydroxylate compound complex obtained from operation B), as follows: 0.01 mol. of albumin with the complex obtained from 2.5 mol. of Mohr's salt and 0.5 mol. of polyhydroxylate compound.

The mixture is heated for one hour at 40°C.
C₂) The complex is precipitated by gradually adding acetic acid to the solution of step C₁ while stirring.

The addition of acetic acid is continued until the solution reaches a pH of between 5.0 and 5.5.
C₃) The precipitate is separated by means of filtration and washed with a 1% solution of acetic acid until the reaction of the iron and the proteins disappears.

The precipitate is dissolved in an aqueous solution (pH 8) containing 10% of glycerol, and the complex is re-precipitated by means of the addition of ammonium sulphate with a degree of saturation of 0.28; finally, the precipitate is filtered and washed with ammonium sulphate having the same degree of saturation.

The precipitate is re-dissolved in an aqueous solution of pH 8 containing 10 wt% of glycerol, and the solution is purified by means of ultrafiltration with molecular fractionation of 20,000 until the reaction of the sulphates disappears.
C₄) The final solution of step C₃ is concentrated to reach approximately 5% of iron and lyophilized in order to obtain the Fe(III)-polyhydroxylate compound-ovalbumin complex in the form of a powder.

The complexes according to the present invention, examined by means of Electron Paramagnetic Resonance (EPR), are found to consist of a core of non-ionic polynuclear iron to which is associated the ovalbumin by means of coordinate bonds reinforced by ionic bonds through the mediation of the polyhydroxylate compound.

This structure presents paramagnetic analogies with ferritin extracted from bovine or equine spleen.

The complexes have the following chemical characteristics:
- Total iron from 135 to 185 mg/g
- Organic iron 97% of total iron
- Organic nitrogen from 80 to 95 mg/g

The anti-anaemic activity of said complexes was pharmacologically verified on sideropenic anaemia induced in rats by means of a diet consisting exclusively of bread and milk (according to Morrith's method).

After approximately 16 weeks, this diet produces an anaemic state in rats, characterized by a drop in haemoglobin (Hb) values of approximately 60%, determined by taking a blood sample from the caudal vein.

The administration of the complex according to the present invention in the form of a hydroglycerine solution at the dose of 1 mg/day/animal added to the milk induced a rise in Hb values in the subsequent 16 weeks of observation, as against a maintenance of the hypochromic levels in the control group.

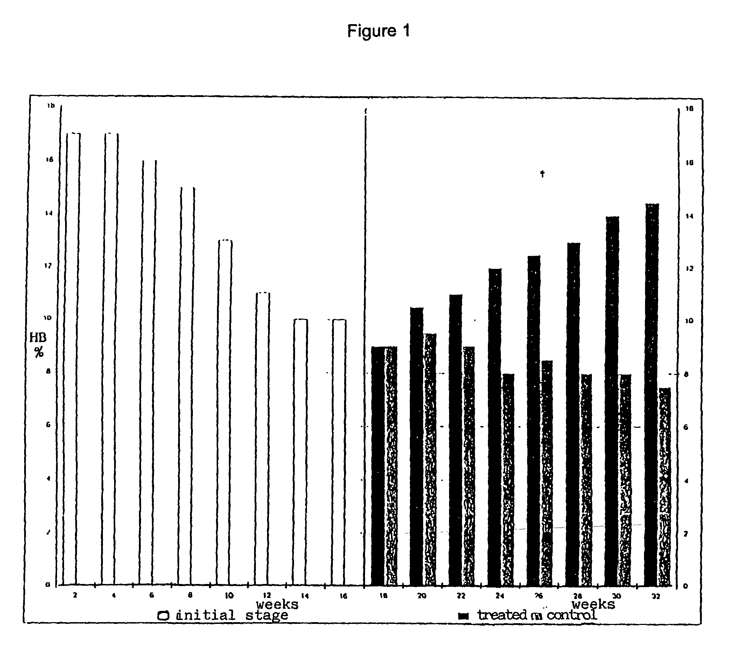

The results of the experimentation are tabulated in Table 1 below and illustrated in Figure 1.

### Table 1

Induced anaemia: microcytic hypochromic anaemia resulting from an iron-deficient diet
Animals: Charles River male rats (mean weight, 384 g)
Diet: bread and milk
Haemoglobin test: Drabkin-Austin test on sample taken from caudal vein every two weeks (Hb%)
Duration of experimentation: 32 weeks
Initial Hb values: Hb 17%
Hb values at 16th week: Hb 9% ± 0.24% (mean weight 228 g)
Survival rate: 97%
Treated group: dose of 1 mg/kg/day (approx. 300 mcg Fe⁺⁺⁺) of Ferri-Manitol-Ovalbumin of example 1 diluted in milk for 16 weeks
Control group: iron-deficient diet
Results at 32nd week
Treated group: Hb 14.5% ± 0.37% (mean weight 273 g)
Control group: Hb 7.5% ± 0.18% (mean weight 225 g)
Survival rate of treated rats: 95%
Survival rate of control rats: 42%

The administration of the complex induced a positive variation in the concentration of haemoglobin, with a net improvement in the general state of the animals in the form of body weight increase and an improvement of the digestive function, with disappearance of the diarrhoeal state.

Thanks to this activity, the complexes according to the present invention may be used as active substances in the preparation of pharmaceutical compositions for the treatment of sideropenic anaemia in man and as a maintenance factor in dietary preparations.

In the treatment of sideropenic anaemia in man, the oral administration of complexes of 80 to 130 mg of Fe per day are envisaged.

The examples that follow are provided for the purpose of illustrating the invention.

### Example 1: Preparation of Ferri-Mannitol-Ovalbumin

A) 0.6 kg of albumin, in the form of flakes, are dissolved in 15 litres of water at a temperature of 10°C under slow stirring for 12 hours, and H₂SO₄ is added up to pH 4. To a markedly turbid solution are added 3.8 kg of ammonium sulphate in small doses (degree of saturation 32%). After being left to rest overnight, the solution is decanted and filtered. A second dose of ammonium sulphate is added to the filtrate until the concentration of 40% is reached (degree of saturation 52%). The precipitated ovalbumin is collected on the filter and washed with 40% ammonium sulphate solution until any foreign proteins have disappeared. The ovalbumin is dissolved in distilled water at the concentration of 2% and then undergoes filtration. The clear solution is purified by ultrafiltration with molecular fractionation of 20,000 until there is total elimination of both organic impurities and ammonium sulphate by means of repeated washings and concentrations.
   A concentration of 5% is finally obtained, and the solution is brought up to a pH of 9 with NaOH solution.
   The yield is approximately 450 g in 10 litres.
B) 1.05 kg of ferrous ammonium sulphate (Mohr's salt) are dissolved in 20 litres of water cooled to 10°C. After dissolution, 100 g of pure mannitol are added, and after 30 minutes approximately 23 ml of 36% hydrogen peroxide are added while the solution is kept stirred for 15 minutes.

100 ml of 20% sodium hydroxide is added to the solution, which is kept stirred and heated up to 80°C for one hour until the reddish-brown complex consisting of Fe(III)-mannitol is completely dissolved. After 1:1 dilution with water, the solution is kept stirred continuously overnight. After filtration, the clear solution is purified using a molecular fractionation (1000) column, washed until a colourless and concentrated effluent up to approximately 10 litres is obtained.

The homogeneous and stable structure of the Fe(III)-mannitol complex becomes evident from the behaviour in the interval between pH 9 and pH 12 with dissolution at alkaline pH, precipitation between pH 6 and pH 7 and redissolution at acidic pH. Electrophoresis in cellulose acetate reveals the displacement of the complex towards the acidic pH anode and migration towards the alkaline buffer cathode. The Electron Paramagnetic Resonance (EPR) spectrum, determined at the frequency of 9,000 MHz and at the temperature of 123° Kelvin indicates the presence of a system with a number of paired paramagnetic centres, i.e., a polynuclear system of Fe³⁺ with high spin. The weak signals at 3,125 gauss are due to the presence of radicals resulting from the complexity of the matrix.
C) The solution containing the Fe(III)-mannitol complex is mixed with the solution containing ovalbumin.

The solution obtained is heated up to 40°C for one hour and then cooled to 25°C. By means of addition of acetic acid down to a pH of 5, the complex consisting of Fe(III)-mannitol-ovalbumin precipitates.

The precipitate is separated by filtration and washed with a 1% solution of acetic acid.

The precipitate is dissolved in an aqueous solution containing 10% of glycerol at pH 8 and then re-precipitated by addition of ammonium sulphate to a degree of saturation of 28%.

After 12 hours, the precipitate is separated by filtration and then re-dissolved in an aqueous solution at pH 8.

This solution is purified through a molecular fractionation (20,000) column until the sulphates disappear, and then concentrated to 3% of total iron.

This solution is lyophilized to obtain the complex in the form of a reddish-brown powder having the following chemical characteristics:
- Total iron 145.5 mg/g
- Organic iron 97% of total iron
- Organic nitrogen 85.0 mg/g

The complex contains 256 moles of Fe(III) (as ferric hydroxide), 51 moles of mannitol (C₆H₁₄O₆) per mole of ovalbumin. The stoichiometric composition is thus found to be the following:

[Fe(OH)₃]_{256•}[(C₆H₁₄O₆)₅₁]• Ovalbumin

with a molecular mass of 80 Kd.

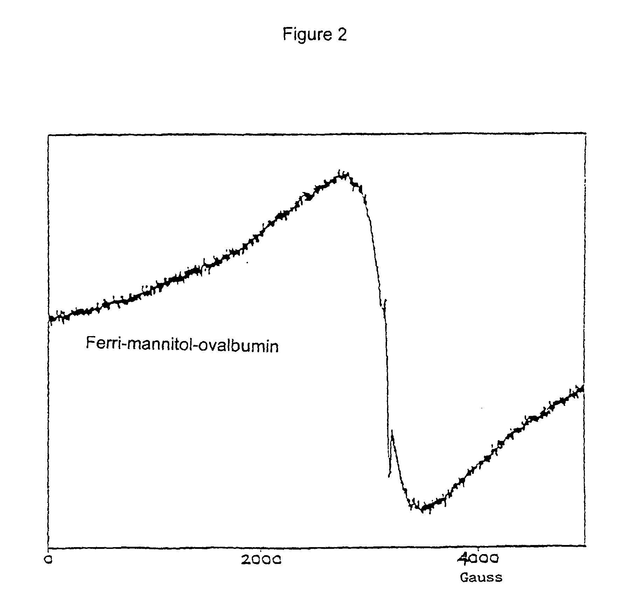

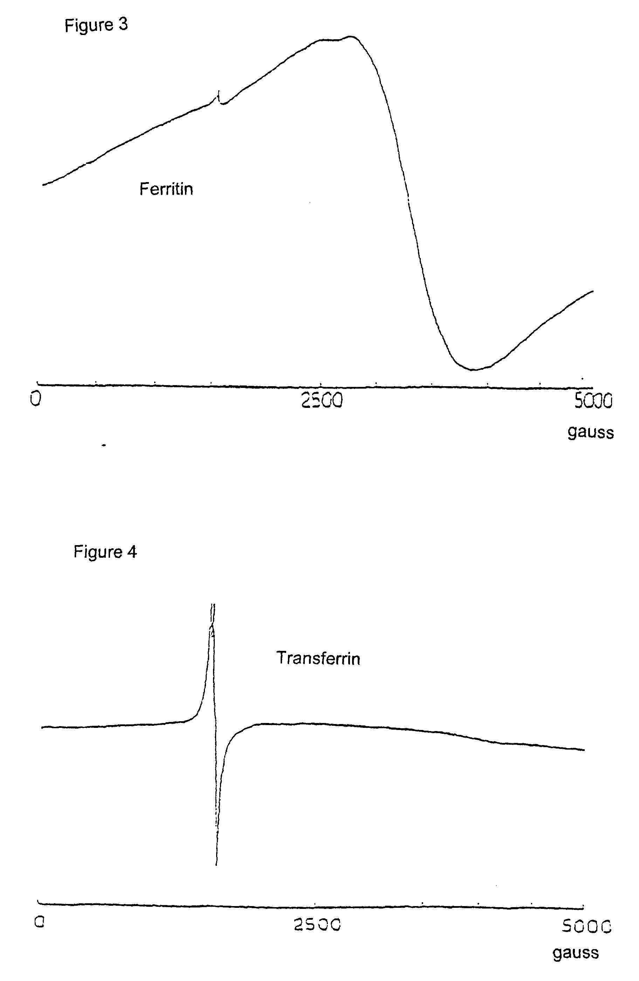

The stability and homogeneity of the complex are evidenced by numerous characteristics, such as:
- single-band uniform migration in electrophoresis reactive to iron and to the protein according to the specifications of the product;
- higher mobility of the complex as compared to ovalbumin in electrophoresis on cellulose acetate;
- close correspodence on SDS-PAGE in gradient between detection of the iron and that of the protein;
- immediate solubility at neutral or alkaline pH, followed by precipitation between pH 5 and pH 6 and re-dissolution at lower pH values;
- stability under heating up to 90°C at the concentration of 4 mg/ml at pH 8, with absence of coagulation phenomena;
- stability of the complex at various acidic pH values;
- absence of denaturation phenomena after precipitation with hydrophilic solvents and with saline solutions (sodium chloride, ammonium sulphate);
- Visible-UV absorption spectra, with absence of maxima in the visible range, with appearance of a well-defined band at 270 nm;
- HPLC analysis with detection of the homogeneous organic complex;
- EPR spectrum (fig. 2) on Varian E/109 working at 9 GHZ at the temperature of 123° Kelvin, with intense and very broad signal around 3,000 gauss, which indicates the presence of a system of multiple paired paramagnetic centres i.e., a polynuclear system of iron atoms with high spin, like the one present in the core of ferritin (Fig. 3) and unlike other structures in which the iron is not in a polynuclear form but in a form co-ordinated to amino acids (transferrin) (Fig. 4) and presents a very intense signal around 1500 gauss.

The pharmacological characteristics of the complex are given in the detailed description of the invention.

### Example 2

Example 1 was repeated with the difference that sorbitol was used instead of mannitol.

The Fe(III)-sorbitol-ovalbumin complex is obtained with analytical and pharmacological characteristics similar to those of Example 1.

### Example 3

Example 1 was repeated with the difference that fructose was used instead of mannitol.

The Fe(III)-fructose-ovalbumin complex is obtained with analytical and pharmacological characteristics similar to those of Example 1.

## Claims

1. Process for the preparation of complexes consisting of Fe(III), a polyhydroxylate compound and ovalbumin, where the said polyhydroxylate compound is chosen from among the group consisting of mannitol, sorbitol and fructose, **characterized by** the following operations:
A) Preparation of an aqueous solution of ovalbumin starting from an aqueous solution of albumin, in which the said albumin solution is treated with ammonium sulphate with a degree of saturation of 32%, to obtain a precipitate that is eliminated and a solution that is treated with ammonium sulphate with a 52% degree of saturation, thus obtaining the precipitation of the ovalbumin, which is re-dissolved in water, purified, concentrated at approximately 5%, and to which is added NaOH to obtain a pH of 9;
B) Preparation of a solution of a complex consisting of Fe(III) and a polyhydroxylate compound in which to an aqueous solution of Mohr's salt is added the polyhydroxylate compound, and subsequently H₂O₂ is added to oxidize Fe(II) to Fe(III), and NaOH is added to the solution obtained up to a pH of 10.5;
C) Preparation of the Fe(III)-polyhydroxylate compound-ovalbumin complex by mixing the solutions obtained as described in points A) and B) and precipitating the complex by adding acetic acid up to a pH of between 5.0 and 5.5, followed by purification.

2. Process according to Claim 1, **characterized in that** the said albumin solution which is treated with ammonium sulphate with degree of saturation 32% has a concentration of between 2 and 5%, a temperature of between 5 and 10°C, and a pH of between 3 and 5.

3. Process according to Claim 1, **characterized in that** the said purification of the ovalbumin solution is carried out via filtration followed by ultrafiltration with molecular fractionation of 20,000.

4. Process according to Claim 1, **characterized in that** the said aqueous solution of Mohr's salt to which is added the said polyhydroxylate compound has a concentration of between 3 and 5.25 wt% and a temperature of between 10 and 15°C.

5. Process according to Claim 1, **characterized in that** the said polyhydroxylate compound is added to the said solution of Mohr's salt in the quantity of 0.5 mol. per 2.5 mol. of Mohr's salt.

6. Process according to Claim 1, **characterized in that** the said addition of H₂O₂ is made in the proportion of 0.25 mol. per 2.5 mol. of Mohr's salt.

7. Process according to Claim 1, **characterized in that** the said mixing of the solutions obtained as per points A) and B) is made by mixing 0.01 mol. of albumin with the complex obtained from 2.5 mol. of Mohr's salt and 0.5 mol. of polyhydroxylate compound.

8. Process according to Claim 1, **characterized in that** the said purification of the precipitated compound is made by means of dissolution of the precipitate in an aqueous solution of pH 8 containing 10 wt% of glycerol, re-precipitation by means of addition of ammonium sulphate with a degree of saturation of 28%, followed by filtration and washing, and finally re-dissolution in aqueous solution (pH 8) containing 10 wt% of glycerol, and ultrafiltration with molecular fractionation of 20,000.

9. Organic complexes of Fe(III) having high absorption capacity, consisting of Fe(III), a polyhydroxylate compound and ovalbumin.

10. Complexes according to Claim 9, **characterized in that** the said polyhydroxylate compound is chosen from among the group consisting of mannitol, sorbitol and fructose.

11. Complexes according to Claim 9, **characterized in that** the said complexes have a total iron content of from 135 to 185 mg/g, a content of organic iron of 97% of the total iron, and a content of organic nitrogen of from 80 to 95 mg/g.

12. Complexes according to Claim 9, **characterized in that** in the visible-UV absorption spectrum, absence of maxima is noted in the visible range, with appearance of a well-defined band at 270 nm, and **in that** in the Electron Paramagnetic Resonance (EPR) spectrum at 9 GHZ and at the temperature of 123° Kelvin, an intense and very broad signal is noted at around 3,000 gauss.

13. Use of complexes according to Claim 9 as active substances in the preparation of pharmaceutical compositions for the treatment of sideropenic anaemia and as a maintenance factor in dietary preparations.

## Patentansprüche

1. Verfahren zur Herstellung von Komplexen, die aus Fe(III), einer Polyhydroxylatverbindung und Ovalbumin bestehen, worin die Polyhydroxylatverbindung aus der Gruppe ausgewählt ist, die aus Mannit, Sorbit und Fructose besteht, **gekennzeichnet durch** die folgenden Arbeitsgänge:
(A) Herstellung einer wässrigen Ovalbuminlösung, ausgehend von einer wässrigen Albuminlösung, worin die Albuminlösung mit Ammoniumsulfat mit einem Sättigungsgrad von 32 % behandelt wird, um eine Ausfällung, die verworfen wird, und eine Lösung zu erhalten, die mit Ammoniumsulfat mit einem Sättigungsgrad von 52 % behandelt wird, um **dadurch** die Ausfällung des Ovalbumins zu erhalten, das erneut in Wasser gelöst, gereinigt, auf ca. 5 % aufkonzentriert und zu dem NaOH zum Erhalt eines pH-Werts von 9 gegeben wird;
(B) Herstellung einer Lösung aus einem Komplex, der aus Fe(III) und einer Polyhydroxylatverbindung besteht, worin die Polyhydroxylatverbindung zu einer wässrigen Lösung aus Mohrschem Salz gegeben wird und anschliessend H₂O₂ zur Oxidation von Fe(II) zu Fe(III) hinzugegeben wird, und NaOH zur erhaltenen Lösung bis zu einem pH-Wert von 10,5 hinzugegeben wird.
(C) Herstellung des Fe(III)-Polyhydroxylatverbindung-Ovalbumin-Komplexes **durch** Vermischen der wie unter den Punkten (A) und (B) beschrieben erhaltenen Lösungen und Ausfällen des Komplexes **durch** Zugabe von Essigsäure auf einen pH-Wert zwischen 5,0 und 5,5, gefolgt von Reinigung.

2. Verfahren gemäss Anspruch 1, **dadurch gekennzeichnet, dass** die Albuminlösung, die mit Ammoniumsulfat mit einem Sättigungsgrad von 32 % behandelt wird, eine Konzentration zwischen 2 und 5 %, eine Temperatur zwischen 5 und 10°C und einen pH-Wert zwischen 3 und 5 hat.

3. Verfahren gemäss Anspruch 1, **dadurch gekennzeichnet, dass** die Reinigung der Ovalbuminlösung über eine Filtration, gefolgt von Ultrafiltration mit einer molekularen Fraktionierung von 20.000 durchgeführt wird.

4. Verfahren gemäss Anspruch 1, **dadurch gekennzeichnet, dass** die wässrige Lösung des Mohrschen Salzes, zu dem die Polyhydroxylatverbindung gegeben wird, eine Konzentration zwischen 3 und 5,25 Gew.% und eine Temperatur zwischen 10 und 15°C hat.

5. Verfahren gemäss Anspruch 1, **dadurch gekennzeichnet, dass** die Polyhydroxylatverbindung zu der Lösung des Mohrschen Salzes in einer Menge von 0,5 mol pro 2,5 mol des Mohrschen Salzes gegeben wird.

6. Verfahren gemäss Anspruch 1, **dadurch gekennzeichnet, dass** die Zugabe von H₂O₂ in einem Verhältnis von 0,25 mol auf 2,5 mol des Mohrschen Salzes durchgeführt wird.

7. Verfahren gemäss Anspruch 1, **dadurch gekennzeichnet, dass** das Vermischen der gemäss den Punkten (A) und (B) erhaltenen Lösungen durch Vermischen von 0,01 mol Albumin mit dem aus 2,5 mol Mohrsches Salz und 0,5 mol Polyhydroxylatverbindung erhaltenen Komplex erfolgt.

8. Verfahren gemäss Anspruch 1, **dadurch gekennzeichnet, dass** die Reinigung der ausgefällten Verbindung mittels Auflösung der Ausfällung in einer wässrigen Lösung mit pH 8, die 10 Gew.% Glycerin enthält, erneute Ausfällung mittels Zugabe von Ammoniumsulfat mit einem Sättigungsgrad von 28 %, gefolgt von Filtration und Waschen, und schliesslich erneuter Auflösung in wässriger Lösung (pH 8), die 10 Gew.% Glycerin enthält, und Ultrafiltration mit einer molekularen Fraktionierung von 20.000 erfolgt.

9. Organische Komplexe von Fe(III) mit hoher Absorptionskapazität, die aus Fe(III), einer Polyhydroxylatverbindung und Ovalbumin bestehen.

10. Komplexe gemäss Anspruch 9, **dadurch gekennzeichnet, dass** die Polyhydroxylatverbindung aus der Gruppe ausgewählt ist, die aus Mannit, Sorbit und Fructose besteht.

11. Komplexe gemäss Anspruch 9, **dadurch gekennzeichnet, dass** die Komplexe einen Gesamteisengehalt von 135 bis 185 mg/g, einen Gehalt an organischem Eisen von 97 % des Gesamteisens und einen Gehalt an organischem Stickstoff von 80 bis 95 mg/g aufweisen.

12. Komplexe gemäss Anspruch 9, **dadurch gekennzeichnet, dass** im UV-VIS-Absorptionsspektrum die Abwesenheit von Maxima im sichtbaren Bereich neben dem Erscheinen einer wohldefinierten Bande bei 270 nm festgestellt wird, und dass im Elektronen-Paramagnetische Resonanz-(EPR)-Spektrum bei 9 GHz und bei einer Temperatur von 123K ein intensives und sehr breites Signal bei ca. 3.000 Gauss festgestellt wird.

13. Verwendung von Komplexen gemäss Anspruch 9 als Wirkstoffe in der Herstellung von pharmazeutischen Zusammensetzungen zur Behandlung von sideropenischer Anämie und als Aufrechterhaltungsfaktor in Nahrungszubereitungen.

## Revendications

1. Procédé pour la préparation de complexes constitués de Fe(III), d'un composé polyhydroxylé et d'ovalbumine, dans lequel ledit composé polyhydroxylé est choisi dans le groupe consistant en mannitol, sorbitol et fructose, **caractérisé par** les opérations suivantes :
(A) préparation d'une solution aqueuse d'ovalbumine à partir d'une solution aqueuse d'albumine, dans laquelle ladite solution d'albumine est traitée avec du sulfate d'ammonium ayant un degré de saturation de 32%, pour obtenir un précipité qui est éliminé et une solution qui est traitée avec du sulfate d'ammonium ayant un degré de saturation de 52%, de façon à obtenir la précipitation de l'ovalbumine, qui est redissoute dans de l'eau, purifiée, concentrés à environ 5%, et additionnée de NaOH pour obtenir un pH de 9 ;
(B) préparation d'une solution d'un complexe constitué de Fe(III) et d'un composé polyhydroxylé, dans laquelle une solution aqueuse de sel de Mohr est additionnée du composé polyhydroxylé, et puis ensuite de H₂O₂ pour oxyder le Fe(II) en Fe(III), et du NaOH est ajouté à la solution obtenue jusqu'à obtention d'un pH de 10,5 ;
(C) préparation du complexe de Fe(III), de composé polyhydroxylé et d'ovalbumine par mélange des solutions obtenues de la façon qui est décrite dans les points (A) et (B) et par précipitation du complexe par addition d'acide acétique jusqu'à un pH compris entre 5,0 et 5,5, puis par purification.

2. Procédé suivant la revendication 1, **caractérisé en ce que** ladite solution d'albumine qui est traitée avec du sulfate d'ammonium ayant un degré de saturation de 32% a une concentration comprise entre 2 et 5%, une température comprise entre 5 et 10°C, et un pH compris entre 3 et 5.

3. Procédé suivant la revendication 1, **caractérisé en ce que** ladite purification de la solution d'ovalbumine est effectuée par filtration, suivie d'une ultrafiltration avec un seuil de fractionnement moléculaire de 20.000.

4. Procédé suivant la revendication 1, **caractérisé en ce que** ladite solution aqueuse de gel de Mohr à laquelle est ajouté ledit composé polyhydroxylé a une concentration comprise entre 3 et 5,25% en poids et une température comprise entre 10 et 15°C.

5. Procédé suivant la revendication 1, **caractérisé en ce que** ledit composé polyhydroxylé est ajouté à ladite solution de sel de Mohr en une quantité de 0,5 mole pour 2,5 moles de sel de Mohr.

6. Procédé suivant la revendication 1, **caractérisé en ce que** ladite addition de H₂O₂ est faite à raison de 0,25 mole pour 2,5 moles de sel de Mohr.

7. Procédé suivant la revendication 1, **caractérisé en ce que** ledit mélange des solutions obtenues selon les points (A) et (B) est fait par mélange de 0,01 mole d'albumine avec le complexe obtenu à partir de 2,5 moles de sel de Mohr et de 0,5 mole de composé polyhydroxylé.

8. Procédé suivant la revendication 1, **caractérisé en ce que** ladite purification du composé précipité est faite par dissolution du précipité dans une solution aqueuse de pH 8 contenant 10% en poids de glycérol, par reprécipitation par addition de sulfate d'ammonium ayant un degré de saturation de 28%, puis par filtration et lavage, et finalement par redissolution dans une solution aqueuse (pH 8) contenant 10% en poids de glycérol, et par ultrafiltration avec un seuil de fractionnement moléculaire de 20.000.

9. Complexes organiques de Fe(III) ayant une haute capacité d'absorption, constitué de Fe(III), d'un composé polyhydroxylé et d'ovalbumine.

10. Complexes suivant la revendication 9, **caractérisés en ce que** ledit composé polyhydroxylé est choisi dans le groupe consistant en mannitol, sorbitol et fructose.

11. Complexes suivant la revendication 9, **caractérisés en ce que** lesdits complexes ont une teneur totale du fer total et une teneur en azote organique de 80 à 95 mg/g.

12. Complexes suivant la revendication 9, **caractérisés en ce que** dans le spectre d'absorption visible et UV, il est observé une absence de maxima dans la gamme du visible, avec apparition d'une bande bien définie à 270 nm, et **en ce que** dans le spectre de résonance paramagnétique électronique (EPR) à 9 GHz et à la température de 123 K, il est observé un signal intense et très large aux environs de 3.000 gauss.

13. Utilisation de complexes, suivant la revendication 9 en tant que substances actives dans la préparation de compositions pharmaceutiques pour le traitement de l'anémie sidéropénique et en tant que facteur d'entretien dans des préparations diététiques.
